# EUROPEAN PATENT SPECIFICATION

(11) **EP 0 348 136 B1**
(45) Date of publication and mention of the grant of the patent: **04.08.1993**
(21) Application number: 89306198.6
(22) Date of filing: 20.06.1989
(51) Int. Cl.: A61M 25/00

(54) **Catheters and methods of manufacture**
Katheter und Methoden zur Herstellung
Catheters et Méthodes de fabrication

(30) Priority: 22.06.1988 GB 8814898
(43) Date of publication of application: 27.12.1989
(73) Proprietor: EVANS, John Martin, Oaksmere Appleton Abingdon Oxfordshire OX13 5JS (GB)
(72) Inventor: EVANS, John Martin, Oaksmere Appleton Abingdon Oxfordshire OX13 5JS (GB)
(74) Representative: Flint, Jonathan McNeill

(56) References cited:
- EP-A- 0 125 844
- EP-A- 0 250 891
- US-A- 4 801 297

## Description

This invention relates to epidural catheters of the kind of comprising a flexible plastics tube having a plurality of elongate slits through the wall of the tube at its patient end, the slits being disposed around the circumference.

Epidural catheters are used to convey an anaesthetic agent to the epidural space of a patient. The catheter conventionally comprises a small bore, flexible, plastics tube the tip of which is introduced to the epidural space through a hollow metal needle that is subsequently removed.

The catheter must be sufficiently rigid to enable it to be manipulated and introduced without kinking but it must also be sufficiently flexible to enable it to be diverted, on insertion to the epidural space, without puncturing veins, dura or other obstacles.

The patient end of an epidural catheter usually opens in one of two different ways. It either has an axial opening at its tip, or a closed tip with several eyes through the wall of the catheter spaced apart from one another along the catheter in a region close to the tip, such as described in US 3,885,561. In the catheter described in this US patent, the eyes are in the form of short slits spaced from one another at the patient end.

Previous epidural catheters suffer from various problems. Firstly, it has not been possible, heretofore, to achieve a low-cost catheter that is sufficiently flexible to prevent any risk of puncturing whilst also preventing the catheter kinking and ensuring occlusion-free flow along the catheter. One form of catheter that attempts to address this problem comprises a tightly wound helical coil of wire embedded in the wall of the catheter to give it radial strength. The coils of the wire are opened out at the patient end to render it more flexible. This form of catheter is, however, expensive to make.

A second problem arises because, if the tip of the catheter does penetrate, for example, the dura, this may initially go undetected. It is usual practice for the anaesthetist to aspirate through the catheter to check for the presence of cerebro-spinal fluid (indicating penetration of the dura) or blood (indicating penetration of a vein). If this fails to indicate incorrect insertion, the anaesthetist will usually inject a small test dose (typically, about 2 cc) of anaesthetic agent which may also include adrenalin. If the catheter lies in the dura or a vein, this will cause a rapid, readily noticeable effect on the patient, indicating false insertion, without fatal consequences. Any blockage of the catheter will be apparent by increased resistance of the syringe used to aspirate or introduce the test dose. However, if the cannula has several eyes, it may be that only one of these is blocked by being located in the dura, the other eyes being unblocked and located in the epidural space. It can be difficult to detect such a situation since flow can occur out of the catheter through the two unblocked eyes. Where anaesthetic agent is only injected slowly it may flow out of the patient end of the cannula entirely through the unblocked eyes, without harm to the patient. Where, however, a higher pressure, top-up dose, is given, the pressure may be sufficient to allow some of the drug to flow into the dura through the patient-end eye with often fatal results.

Those catheters opening through only the patient end tip, are more likely to be occluded. Futhermore, the open end of the such catheters tends to encourage jetting at the tip.

It is an object of the present invention to provide a catheter that can be made at low cost with a more flexibe tip so as thereby to reduce the risk of puncturing a vein or the dura. It is another object of the present invention to provide a method of making such a catheter.

According to one aspect of the present invention there is provided an epidural catheter of the above-specified kind, characterised in that a substantial part at least of each slit extends along the same region along the length of the catheter at its patient end such that flexibility of the catheter is thereby increased over the region.

The slits preferably extend parallel to one another and to the longitudinal axis of the tube.

According to another aspect of the present invention there is provided an epidural catheter comprising a flexible plastics tube having an elongate slit through the wall of the tube at its patient end, characterised in that the slit extends helically about the tube such that the flexibility of the patient end of the catheter is thereby increased.

The patient end tip of the catheter is preferably closed. The or each slit may be formed without removal of material. The catheter may include an aperture located rearwardly of the slits.

According to a further aspect of the present invention there is provided a method of making an epidural catheter comprising the steps of: providing a flexible plastics tube with an open patient end tip, forming a plurality of slits through the wall of the tube each slit extending from the open patient end tip along substantially the same region along the length of the tube and being separated from one another by segments of the tube, and closing the patient end tip of the tube by joining the patient end of the segments while leaving the slits open along a major part of their length such that the flexibility of the catheter is increased over a region at the patient end of the catheter.

The slits are preferably made by pushing the patient end of the tube along its length over an arrangement of crossed blades. The patient end of the segments are preferably subsequently joined together by end forming in a heated die.

An epidural catheter and a method of manufacture of the catheter, in accordance with the present invention, will now be described, by way of example, with reference to the accompanying drawings, in which:
- Figure 1: is a side elevation view of the catheter;
- Figure 2: is an enlarged transverse cross-section of the catheter along the line II - II in Figure 1;
- Figure 3: illustrates the catheter in use;
- Figures 4 to 6: are side views of the patient ends of alternative catheters;
- Figures 7 to 9: illustrate steps in the manufacture of a form of catheter;
- Figures 10 and 11: illustrate steps in the manufacture of another form of catheter; and
- Figure 12: is a side elevation view of a alternative form of catheter.

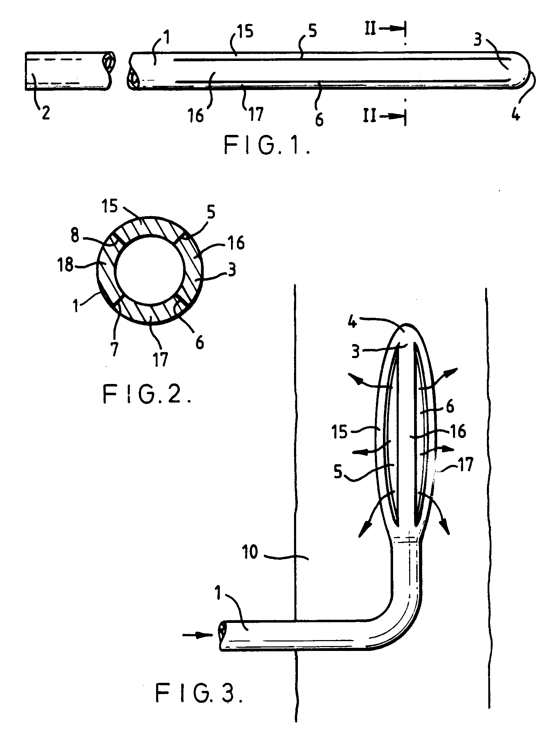

With reference first to Figures 1 and 2, the epidural catheter comprises a tube 1 of a flexible plastics material, such as nylon, and is about 850 mm long, being circular in section with an external diameter of 1 mm and an internal diameter of 0.58 mm. The machine end 2 of the catheter is square, plain and open enabling it to be joined to any conventional epidural connector.

The patient end 3 of the catheter has a smoothly rounded closed tip 4 and has four slits 5 to 8 formed through the wall of the tube 1. The slits 5 to 8 are formed without removal of any material from the wall of the tube 1 and are equally spaced from one another around the catheter. The slits 5 to 8 are 10 mm long and each extend parallel to one another and to the catheter longitudinal axis from just rearwardly of the tip 4. The slits 5 to 8 thereby extend along the same region along the length of the catheter and define four segments 15 to 18 between them around the catheter.

With reference now also to Figure 3, the patient end 3 of the catheter is introduced to the epidural space 10 in the usual way. When fluid is supplied to the machine end 2 of the catheter it is free to flow out of the patient end 3 through the slits 5 to 8. The length of the slits 5 to 8 and the nature of the material from which the catheter is made are such as to ensure that some at least of the slits are sufficiently open to allow free flow of injected fluid out of the catheter. The pressure of fluid within the catheter may also help to open the slits 5 to 8 slightly. The slits 5 to 8 increase the flexibility at the patient end 3 of the catheter so that it is more easily deflected if it should meet an obstruction on insertion. In this way, the risk of veinepuncture and dural puncture are substantially reduced.

It will be appreciated that fluid will be delivered through the slits 5 to 8 at very much the same location along the catheter, regardless of injection pressure. The catheter is also less likely to be occluded than those catheters having a single opening. Furthermore, dispersal of fluid from the slits 5 to 8 is improved and jetting is avoided. Because the opening of the catheter is of a shorter length than the region over which eyes are located in conventional multiple eye catheters, there is less risk of false test injection result being obtained.

Various modifications are possible within the scope of the present invention. For example, the slits could be formed by removal of material from the wall of the tube.

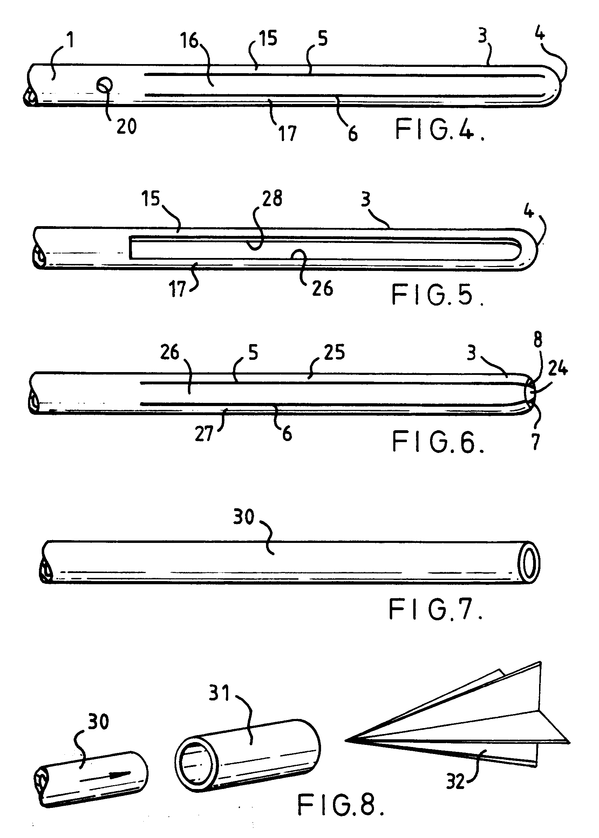

An aperture 20 could be formed in the catheter to the rear, distally, of the slits 5 to 8, as shown in Figure 4. This has the advantage of reducing the risk, when suction is applied to the machine end 2 to aspirate the catheter, of the segments 15 to 18 being sucked inwardly, blocking the bore through the catheter. It also prevents the edges of the four segments 15 to 18 sealing with each other to form a closed ended cylinder.

Figure 5 shows an alternative way in which the catheter can be prevented from closing an aspiration. In this arrangement, two of the segments 16 and 18 have been removed thereby producing enlarged slits 26 and 28.

The tip of the catheter at the patient end 3 could be open in the manner shown in Figure 6. In this arrangement, the slits 5 to 8 extend to the open end 24 of the catheter so that the segments 25 to 28 are free at their patient end. Such an arrangement also reduces the risk of the catheter closing on aspiration.

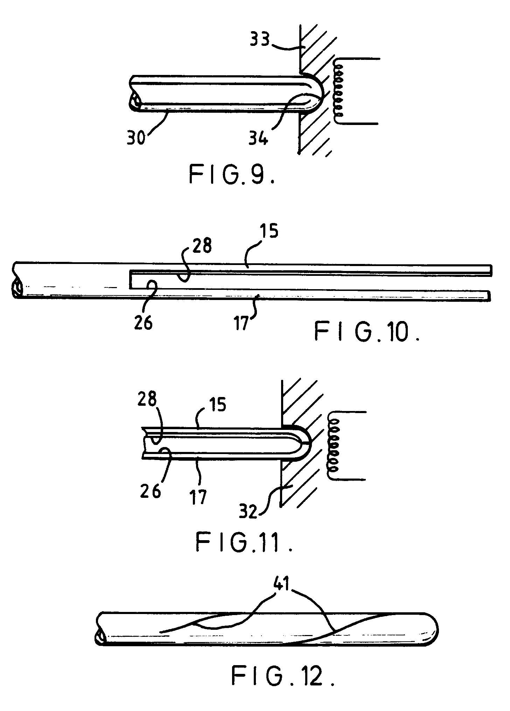

A catheter of the kind shown in Figures 1 to 3 may be made in the manner shown in Figures 7 to 9. A plain square-ended tube 30, of the kind shown in Figure 7, is provided and cut diameterically along a part of its length. This is done by pushing the tube 30 through an outer support tube 31 so that the open end of the tube impinges on a cruciform arrangement 32 of crossed, pointed blades. The four slits 5 to 8 thereby formed extend to the open patient end of the tube 30. The patient end of the tube 30 is closed, as shown in Figure 9, by end forming, such as in a heated die 33 have a rounded concave recess 34 of the desired profile of the finished catheter. The temperature of the die 33 is sufficient to cause softening and flow of the material of the tube so that the ends of the segments 15 to 18 flow into one another and become joined after cooling. It has been found preferable to form the slits before closing the patient end, in the way described above, since this makes it easier to ensure that the slits 5 to 8 extend close to the tip of the catheter than if the slits are produced after closing the end of the catheter.

A catheter having a tip of the kind shown in Figure 5 can be formed in a similar way, as shown in Figures 10 and 11. Following cutting the slits, in the manner described above with reference to Figure 8, two of the segments 16 and 18 are cut away to leave the remaining two segments 15 and 17 separated by enlarged slits 26 and 28. The patient end of the two segments are then bridged together using the heated die 33, as shown in Figure 11. This produces a catheter with a closed patient end and with enlarged slits 26 and 28 extending from close to the patient end.

It will be appreciated that various modifications could be made to the arrangements described above. For example, the slits could be staggered from one another slightly along the length of the catheter providing they overlap substantially.

Instead of having a plurality of slits, a single helical slit 41 extending about the patient end of the catheter could be used, as shown in Figure 12.

## Claims

1. An epidural catheter comprising a flexible plastics tube having a plurality of elongate slits through the wall of the tube at its patient end, the slits being disposed around the circumference of the catheter, characterised in that a substantial part at least of each slit (5 to 8) extends along the same region along the length of the catheter such that flexibility of the catheter is thereby increased over the region.

2. An epidural catheter according to Claim 1, characterised in that the slits (5 to 8) extend parallel to one another and to the longitudinal axis of the tube (1).

3. An epidural catheter comprising a flexible plastics tube having an elongate slit through the wall of the tube at its patient end, characterised in that the slit (41) extends helically about the tube such that flexibility of the patient end of the catheter is thereby increased.

4. An epidural catheter according to any one of the preceding claims, characterised in that the patient end tip (4) of the catheter is closed.

5. An epidural catheter according to any one of the preceding claims, characterised in that the or each slit (5 to 8 or 41) is formed without removal of material.

6. An epidural catheter according to any one of the preceding claims, characterised in that the catheter includes an aperture (20) located rearwardly of the slits (5 to 8).

7. A method of making an epidural catheter comprising the steps of: providing a flexible plastics tube (30) with an open patient end tip, forming a plurality of slits (5 to 8) through the wall of the tube (30) each slit extending from the open patient end tip along substantially the same region along the length of the tube (30) and being separated from one another by segmonts (15 to 18) of the tube, and closing the patient end tip of the tube by joining together the patient end of the segments (15 to 18) while leaving the slits (5 to 8) open along the major part of their length such that the flexibility of the catheter is increased over a region at the patient end (3) of the catheter.

8. A method according to Claim 7, characterised in that the slits (5 to 8) are made by pushing the patient end of the tube (30) along its length over an arrangement (32) of crossed blades.

9. A method according to Claim 7 or 8, characterised in that the patient end of the segments (15 to 18) are subsequently joined together by end forming in a heated die (33).

## Patentansprüche

1. Epiduralkatheter mit einem flexiblen Kunststoffschlauch, der an seinem patientenseitigen Ende eine Vielzahl länglicher Schlitze durch seine Wandung aufweist, wobei diese Schlitze entlang des Umfangs des Katheters angeordnet sind**, dadurch gekennzeichnet,** daß mindestens ein erheblicher Teil jedes Schlitzes (5 bis 8) entlang des gleichen Bereichs entlang der Länge des Katheters verläuft, so daß die Flexibilität des Katheters in diesem Bereich erhöht wird.

2. Epiduralkatheter nach Anspruch 1, d**adurch gekennzeichnet,** daß die Schlitze (5 bis 8) parallel zueinander und zur Längsachse des Schlauches (1) verlaufen.

3. Epiduralkatheter mit einem flexiblen Kunststoffschlauch, dessen Wandung an seinem patientenseitigen Ende einen länglichen Schlitz aufweist, **dadurch gekennzeichnet,** daß der Schlitz (41) helikal um den Schlauch herum verläuft, wodurch die Flexibilität des patientenseitigen Endes des Katheters erhöht wird.

4. Epiduralkatheter nach einem der voranstehenden Ansprüche, **dadurch gekennzeichnet,** daß die Spitze (4) des patientenseitigen Endes des Katheters verschlossen ist.

5. Epiduralkatheter nach einem der voranstehenden Ansprüche, **dadurch gekennzeichnet,** daß der bzw. die Schlitze (5 bis 8 oder 41) ohne Entfernung von Material gebildet werden.

6. Epiduralkatheter nach einem der voranstehenden Ansprüche, **dadurch gekennzeichnet,** daß er eine hinter den Schlitzen (5 bis 8) angebrachte Öffnung (20) aufweist.

7. Verfahren zur Herstellung eines Epiduralkatheters, welches folgende Schritte umfaßt: Bereitstellen eines flexiblen Kunststoffschlauchs (30) mit einer offenen Spitze an seinem patientenseitigen Ende, Bilden einer Vielzahl von Schlitzen (5 bis 8) durch die Wand des Schlauches (30), wobei jeder Schlitz von der offenen patientenendseitigen Spitze im wesentlichen entlang des gleichen Bereichs entlang der Länge des Schlauches (30) verläuft und die Schlitze voneinander durch Schlauchsegmente (15 bis 18) getrennt sind, und Schließen der patientenendseitigen Spitze des Schlauches durch Verbinden der patientenseitigen Enden der Segmente (15 bis 18), wobei die Schlitze (5 bis 8) entlang eines Hauptteils ihrer Länge offen belassen werden, wodurch die Flexibilität des Katheters in einem Bereich beim patientenseitigen Ende (3) des Katheters erhöht wird.

8. Verfahren nach Anspruch 7, **dadurch gekennzeichnet,** daß die Schlitze (5 bis 8) hergestellt werden, indem das patientenseitige Ende des Schlauches (30) entlang seiner Länge über eine Anordnung (32) aus gekreuzten Klingen geschoben wird.

9. Verfahren nach einem der Ansprüche 7 oder 8, **dadurch gekennzeichnet,** daß die patientenseitigen Enden der Segmente (15 bis 18) danach durch Endformung in einem beheizten Stempel (33) miteinander verbunden werden.

## Revendications

1. Cathéter épidural comportant un tube flexible en plastique pourvu d'une pluralité de fentes allongées réalisées dans les parois du tube à son extrémité située du côté du patient, les fentes étant disposées sur la circonférence du cathéter, caractérisé en ce qu'au moins une partie substantielle de chaque fente (5 à 8) s'étend le long de la même zone suivant la longueur du cathéter, de sorte que la flexibilité du cathéter est augmentée dans cette zone.

2. Cathéter épidural selon la revendication 1, caractérisé en ce que les fentes (5 à 8) s'étendent parallèlement les unes aux autres et parallèlement à l'axe longitudinal du tube (1).

3. Cathéter épidural comportant un tube flexible en plastique pourvu d'une fente allongée dans la paroi du tube à son extrémité située du côté du patient caractérisé en ce que la fente (41) s'étend de façon hélicoïdale le long du tube de sorte que la flexibilité du catéther à l'extrémité située du côté du patient est augmentée.

4. Cathéter épidural selon l'une quelconque des revendications précédentes, caractérisé en ce que l'extrémité (4) du cathéter située du côté du patient est fermée.

5. Cathéter épidural selon l'une quelconque des revendications précédentes, caractérisé en ce que la ou chaque fente (5 à 8 ou 41) est formée sans enlèvement de matière.

6. Cathéter épidural selon l'une quelconque des revendications précédentes, caractérisé en ce que le cathéter comporte une ouverture (20) située en arrière des fentes (5 à 8).

7. Procédé de fabrication d'un cathéter épidural comportant les étapes consistant à :
- fournir un tube flexible en plastique (30) comportant une extrémité ouverte du côté du patiente,
- réaliser une pluralité de fentes (5 à 8) dans les parois du tube (30), chaque fente s'étendant depuis l'extrémité ouverte située du côté du patient jusqu'à sensiblement une même zone le long de la longueur du tube (30) et étant séparées les unes des autres par des segments (15 à 18) du tube, et
- fermer l'extrémité du tube du côté du patient en joignant ensemble les extrémités situées du côté du patient des segments (15 à 18) tout en maintenant les fentes (5 à 8) ouvertes sur la majeure partie de leur longueur de sorte que la flexibilité du cathéter est augmentée dans une zone proche de l'extrémité côté patient (3) du cathéter.

8. Procédé selon la revendication 7, caractérisé en ce que les fentes (5 à 8) sont réalisées en poussant l'extrémité du tube (30) située du côté du patient dans le sens de sa longueur sur un arrangement (32) de lames croisées.

9. Procédé selon les revendications 7 ou 8, caractérisé en ce que les extrémités situées du côté du patient des segments (15 à 18) sont ensuite jointes ensemble en formant l'extrémité dans une matrice chauffée (33).
